# EUROPEAN PATENT SPECIFICATION

(11) **EP 1 071 483 B1**
(45) Date of publication and mention of the grant of the patent: **13.03.2002**
(21) Application number: 99918093.8
(22) Date of filing: 16.04.1999
(51) Int. Cl.: A61L 27/00

(54) **BONE IMPLANT**
KNOCHENIMPLANTAT
IMPLANT OSSEUX

(30) Priority: 17.04.1998 GB 9808189
(43) Date of publication of application: 31.01.2001
(73) Proprietor: UNIVERSITY COLLEGE LONDON, London NW3 2PF (GB); UNISEARCH LIMITED, Kensington New South Wales, 2033 (AU)
(72) Inventor: REVELL, Peter, Allen, Essex CM14 5ET (GB); HOWLETT, Cameron, Rolfe, Gordon, NSW 2072 (AU)
(74) Representative: Daniels, Jeffrey Nicholas
(86) International application number: GB9901170
(87) International publication number: WO9953971

(56) References cited:
- WO-A-98/17199
- US-A- 4 718 905
- US-A- 4 800 884
- US-A- 4 917 702
- US-A- 5 188 670
- US-A- 5 211 833

## Description

### FIELD OF THE INVENTION

The present invention relates to a bone implant having improved bone ongrowth properties, and a method for treating a bone implant to improve these properties.

### BACKGROUND TO THE INVENTION

A major problem in orthopaedic reconstruction surgery, and in particular in joint replacement surgery, relates to the need to anchor permanently an orthopaedic implant to the skeleton. Usually, whilst bone grows up to the orthopaedic implant, it does not become physically and chemically bonded to the implant.

There are several known methods for achieving anchoring of orthopaedic implants to the skeleton. According to one commonly-known method, a "cement" is used to increase the surface area of the implant thereby to increase its interlock with the bone. Acrylic cements are commonly used for this purpose. However, over extended periods of time, problems are encountered with deterioration of the cement and the consequent loosening of the bone implant from the skeleton.

Another known method for attempting to anchor orthopaedic implants to the skeleton involves designing the implant to have a beaded or porous surface so that bone growing towards the implant will provide an interference fitting between the implant and the ingrowing skeletal tissues (e.g. bone).

A third method for achieving anchoring of an orthopaedic implant into the skeleton involves the use of an implant that includes a coating of a bioactive material such as hydroxyapatite. Bioactive materials are materials that are capable of promoting bone growth onto the implant, and include materials such as fluoroapatite, tricalcium phosphate, glass ionomers and bioactive glass such as Bioglass and AW Glass Ceramic, in addition to hydroxyapatite (HA). Orthopaedic implants having HA coatings currently provide more effective fusion of the implant with the skeleton than other known anchoring techniques.

Since the long term success of orthopaedic implants is highly dependent on the anchoring of the orthopaedic implant to the skeleton, many investigations have been made into other techniques for improving anchoring.

Previously published studies^{1,2,3} have investigated whether modifying the surface chemistry of uncoated structures (some of which are suitable for use as orthopaedic implants) by the incorporation of cations such as magnesium (Mg⁺⁺) enhances the adhesion of human bone-derived cells to these uncoated structures in in vitro studies. Incorporation of cations into ceramic or metallic structures in these previous studies was accomplished by ion beam implantation (embedding), which enables the incorporation of the cations into the ceramic or metallic surface atomic layers without affecting the surface properties thereof. The studies resulted in mixed success.

Accordingly, there still exists a need to develop bone implants having improved bone ongrowth properties, and methods for manufacturing such bone implants.

### SUMMARY OF THE INVENTION

According to the present invention there is provided a bone implant having a surface comprising a bioactive material, said bioactive material having incorporated therein ions from one or more of the groups of the period table consisting of groups IIA, IVA, VIIA and transition elements, said ions being incorporated into or onto the surface of the bone implant by ion beam implantation or cathodic arc deposition, said bioactive material being a material that is capable of promoting bone growth into and/or onto the bone implant, and said ions being capable of improving the bone ongrowth properties.

Preferably the ions are selected from one or more groups of the periodic table consisting of groups IIA, IVB, VIB, VIII, IB, IIB, IVA and VIIA.

Preferably, the bioactive material comprises hydroxyapatite, and preferably the ions are incorporated into the surface of the bone implant by ion beam implantation or cathodic arc deposition.

The inventors have conducted an in vivo study in order to investigate whether the incorporation of ions by ion beam implantation techniques into bioactive material coated metal/metal alloy and/or orthopaedic implants (specifically hydroxyapatite) enhances bone growth onto the orthopaedic implant. The inventors have surprisingly discovered that the addition of particular ions to these coatings greatly enhances bone ongrowth onto the implant when compared with conventional hydroxyapatite (HA) coated metal alloy orthopaedic implants.

The growth of human bone cells onto a surface depends critically on the nature of the surface. Accordingly, whilst it is possible to use methods other than ion beam implantation (embedding) of the ions into the surface of the bone implant (e.g. cathodic arc deposition or formulating the surface of the bone implant to include such ions during formation of the bone implant), ion beam embedding is preferred since this method results in altering the surface chemistry of the surface material without affecting the surface structure and mechanical properties. Accordingly, if another method is used to provide a surface of a bone implant comprising a bioactive material having incorporated therein ions from one or more of the selected groups of the periodic table, care must be taken to ensure that the surface structure and mechanical properties of the surface are as close to the unmodified bioactive material surface properties as possible. The ions should be present in the surface of the bone implant at a level sufficient to achieve enhanced bone ongrowth but not at so great a level as to affect the mechanical and surface properties of the surface.

Preferably, the ions are incorporated into the surface of the bone implant up to a maximum depth of 200nm. Whilst this is the preferred maximum depth of ions, it is possible to implant ions to greater depths, for example 1000nm. However, by implanting ions to these greater depths, there is an increasing risk that the surface and mechanical properties of the hydroxyapatite might be affected, due to the higher temperatures generated to achieve implantation of the ions to these depths. The higher temperatures are reached as a result of the greater energies used in ion beam implantation of the ions into the surface of the bioactive material.

Preferably, the ions are incorporated into the surface of the bone implant up to a maximum depth of 150nm, and preferably at depths ranging up to approximately 100nm.

Preferably, the ions are present in the surface of the bone implant at a level of between 1 x 10¹⁴ and 1 x 10¹⁸ ions per cm² of the surface. These dosage levels correspond to ion beam implantation energies up to approximately 100 kV.

Preferably the ions incorporated into the surface of the bone implant comprise the ions of elements that can form divalent cations, with the exception of silicon. Examples of such ions include the cations of iron, including ferrous and ferric ions, since iron is capable of forming the divalent ferrous cation.

Preferably, the ions incorporated into the surface of the bone implant comprise cations that are involved in metabolic processes in trace amounts.

Preferably the ions incorporated into the surface of the bone implant comprise one or more of the following:
magnesium, calcium, strontium, titanium, chromium, manganese, iron, copper, zinc, silicon and fluorine ions.

Preferably, the ions incorporated into the surface of the bone implant are from one or more of the groups of the periodic table consisting of groups IIA, VIIB, IIB, IVA and VIIA.

More preferably, the ions comprise magnesium, manganese, zinc or silicon ions.

In the case of a bone implant for use in total joint replacements, such as hip replacements, the bone implant will usually comprise a body portion coated with a hydroxyapatite coating. It is preferred that the body portion be formed of a metal or metal alloy, such as cobalt-chrome or titanium alloy. In the case of dental implants, the body portion may comprise a pin formed of a metal alloy coated by a hydroxyapatite coating, which is inserted into the jaw to replace a tooth.

However, it is not always necessary to use a body portion in the bone implant. The present inventors have found that it is also possible to use a bioactive material such as hydroxyapatite without a structural body portion to promote healing in a bone. According to the present invention there is also provided a bone implant wherein the bone implant substantially comprises a bioactive material (preferably hydroxyapatite) and no body portion. In this case, the bone implant is preferably in a granular form. The granular bioactive material embedded with ions of the selected groups of the periodic table can be used in the mending of fractured or defective bones. The granular ion beam implanted hydroxyapatite bone implant material can be packed into the area of the break or defect in the bone. Since this material has excellent bone growth enhancing properties, this material can be advantageously used to speed up the process of bone repair.

According to the present invention there is also provided a method of treating a bone implant having a surface comprising a bioactive material to improve the bone ongrowth properties of the bone implant, comprising subjecting the bone implant to ion beam implantation to thereby incorporate ions from one or more of the groups of the periodic table consisting of groups IIA, IVA, VII A and transition elements into the surface thereof.

Preferably the ions are selected from one or more groups of the periodic table consisting of groups IIA, IVB, VIB, VIII, IB, IIB, IVA and VIIA.

Preferably, the bioactive material comprises hydroxyapatite.

Preferably, the ions are incorporated into the surface of the bone implant at a level of between 1 x 10¹⁴ and 1 x 10¹⁸ ions per cm² of the surface.

### BRIEF DESCRIPTION OF THE DRAWINGS

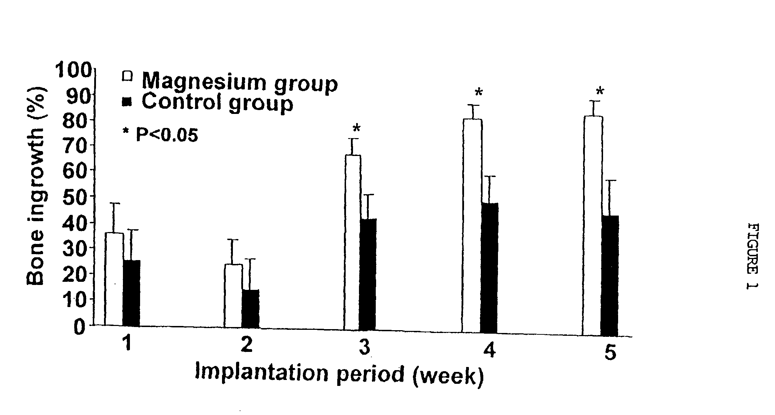

FIGURE 1 shows the percentage of bone ingrowth into an implant slot after 6 weeks of implantation in rabbit femur using an implant according to the invention as compared with a prior art implant.

### DETAILED DESCRIPTION OF THE INVENTION

The present invention will now be described in further detail by reference to the following *in vivo* experimental implantation study.

Cylindrical titanium alloy implants (Ti6Al4V) (4.5 diam x 6mm length) with a slot (2 x 2 x 4 mm) in one side were plasma-spray HA-coated at the bottom of the slot (HA-Ti6Al4V). Identically prepared cylinders were additionally ion beam implanted with Mg⁺⁺ on the HA-coated region using a metal vapour vacuum arc (MEVVA) ion source (Mg-HA-Ti6A14V) (1 x 10¹⁷ ions/cm²Mg⁺⁺). Surgical implantation was performed under general anaesthetic with full sterile precautions into the lateral side of the lower femur of female NZ white rabbits (n=6). A 4.5 mm diameter hole was made using a saline cooled diamond-impregnated trephine and the sterile cylinders (autoclave, 121°C, 15 mins) inserted bilaterally. HA-Ti6Al4V was implanted on the left, Mg-HA-Ti6Al4V on the right. Fluorescent bone labels (tetracycline, calcein blue, calcein green, alizarin red) were administered at weekly intervals and animals killed at 6 weeks. Retrieved femurs were processed in resin and ground sections (30µm) prepared with the implant *in situ* (Exakt System, Hamburg, Germany). The maximum distance that each label had reached in the slots was measured by fluorescence microscopy using an eye-piece graticule and the result expressed as percentage bone ingrowth. The area occupied by new bone after 6 weeks was measured in toluidine blue stained sections using a Quantimet 500 (Leica, Cambridge, UK) and expressed as percentage area of bone formation.

### Results and Discussion

The percentage of bone ingrowth was significantly higher in Mg-HA-Ti6Al4V than in HA-T16A14V implants at 3, 4 and 5 weeks (p<0.05) (Student's 't' test) (see Fig. 1). No significant differences were found at 1 and 2 weeks, though Mg-HA-Ti6Al4V mean values were higher. At 6 weeks, the percentage area of bone formation was significantly greater in the slots with Mg-HA-coating (25.73 ±9.12%, n=5) compared with HA-coating alone (5.86±3.46%, n=5) (p<0.05, Student's 't' test).

These results demonstrate that Mg⁺⁺ ion embedding of an HA-coating increases bone growth into a slot in a Ti6Al4V alloy implant when compared with conventional HA.

As will be appreciated by persons skilled in the art of the invention, whilst the experimental implantation study was conducted using magnesium ion embedding, other ions from the groups of the periodic table consisting of groups IIA, IVA, VIIA and transition elements will also result in enhanced bone formation when compared with conventional HA-coated implants. It will also be appreciated by persons skilled in the art of the invention that ions deleterious to bone mineralisation, such as aluminium (which is implicated in various bone diseases), would not result in enhancement of bone formation. The studies of the present inventors confirm that aluminium and other ions deleterious to bone mineralisation cannot be used in the present invention to increase bone formation.

### References

1. Walsh WR, Zou L, Lefkoe TP, Kelly JC and Howlett CR (1992). Bone cell response to ion implanted silicon wafers. Mat Res Soc Symp 252:213-220.
2. Howlett CR, Evans MDM, Wildish KL, Kelly JC, Fisher LR, Francis GW and Best DJ (1993). The effect of ion implantation on cellular adhesion. Clinical Materials 14:57-64.
3. Howlett CR, Zreiqat H, Noorman H, Evans PA, Dalton BA, O'Dell R, McFarland C and Steele JC (1994). The effect of magnesium ion implantation into alumina upon the adhesion of human bone derived cells. J Materials Science: Materials in Medicine 5:715-722.

## Claims

1. A bone implant having a surface comprising a bioactive material, wherein:
(a) the bioactive material has incorporated therein ions from one or more of the groups of the periodic table consisting of groups IIA, IVA, VIIA and transition elements;
(b) the ions are incorporated into or onto the surface of the bone implant by ion beam implantation or cathodic arc deposition; and
(c) the bioactive material is a material that is capable of promoting bone growth onto the bone implant.

2. The bone implant as claimed in claim 1, wherein the bioactive material comprises hydroxyapatite.

3. The bone implant as claimed in claim 1 or claim 2, wherein the ions are incorporated into the surface atomic layers of the bone implant up to a maximum depth of 200nm.

4. The bone implant as claimed in claim 1 or claim 2, wherein the ions are incorporated into the surface of the bone implant up to a maximum depth of 150 nm.

5. The bone implant as claimed in claim 4, wherein the ions are incorporated into the surface at depths ranging up to approximately 100nm.

6. A bone implant as claimed in any one of the preceding claims wherein the ions are present at a level of between 1 x 10¹⁰ and 1 x 10¹⁸ ions per cm² of the surface.

7. A bone implant as claimed in any one of the preceding claims, wherein the ions are selected from one or more groups of the periodic table consisting of groups IIA, IVB, VIB, VIIB, VIII, IB, IIB, IVA and VIIA.

8. A bone implant as claimed in claim 7, wherein the ions comprise one or more of the following;
magnesium, calcium, strontium, titanium, chromium, manganese, iron, copper, zinc, silicon and fluorine ions.

9. A bone implant as claimed in claim 1, wherein the ions incorporated into the surface of the bone implant are from one or more of the groups of the periodic table consisting of groups IIA, VIIB, IIB, IVA and VIIA.

10. A bone implant as claimed in any one of the preceding claims, wherein the ions comprise magnesium, manganese, zinc or silicon ions.

11. A bone implant as claimed in any one of the preceding claims, comprising a body portion coated with a bioactive material coating.

12. A bone implant as claimed in claim 11, wherein the body portion is formed of a metal or a metal alloy, preferably a titanium alloy.

13. A bone implant as claimed in any one of claims 1 to 10, wherein the bone implant substantially comprises a bioactive material.

14. A bone implant as claimed in claim 13, wherein the bone implant is in granular form.

15. A method of treating a bone implant having a surface comprising a bioactive material to improve the bone ongrowth properties of the bone implant comprising subjecting the bone implant to ion beam embedding thereby to incorporate ions from one or more of the groups of the periodic table consisting of groups IIA, IVA, VIIA and transition elements into the surface.

16. The method as claimed in claim 15, wherein the bioactive material comprises hydroxyapatite.

17. The method as claimed in any one of claim 15 or claim 16, wherein the ions are incorporated into the surface up to a maximum depth of 200nm.

18. The method as claimed in claim 17, wherein the ions are incorporated into the surface up to a maximum depth of 150nm.

19. The method as claimed in claim 18, wherein the ions are incorporated at depths ranging up to approximately 100nm.

20. The method as claimed in any one of claims 14 to 19, wherein the ions are present at between 1 x 10¹⁰ and 1 x 10¹⁸ ions per cm² of the implant surface.

21. The method as claimed in any one of claims 14 to 20, wherein the ions are selected from one or more groups of the periodic table consisting of groups IIA, IVB, VIB, VIIB, VIII, IB, IIB, IVA and VIIA.

22. The method as claimed in claim 21, wherein the ions comprise one or more of the following:
magnesium, calcium, strontium, titanium, chromium, manganese, iron, copper, zinc, silicon and fluorine ions.

23. The method as claimed in claim 21, wherein the ions incorporated into the surface of the bone implant are from one or more of the groups of the periodic table consisting of groups IIA, VIIB, IIB, IVA and VIIA.

24. The method as claimed in claim 23, wherein the ions comprise magnesium, manganese, zinc or silicon ions.

## Patentansprüche

1. Knochenimplantat mit einer Oberfläche, die ein bioaktives Material umfasst, wobei:
(a) das bioaktive Material Ionen aus einer oder mehreren der Gruppen des Periodensystems enthält, welche aus den Gruppen IIA, IVA, VIIA und Übergangselementen bestehen;
(b) die Ionen in oder auf der Oberfläche des Knochenimplantats durch Ionenstrahl-Implantation oder kathodische Lichtbogen-Abscheidung einverleibt werden; und
(c) das bioaktive Material ein Material ist, welches das Knochenwachstum auf das Knochenimplantat hinauf fördern kann.

2. Knochenimplantat nach Anspruch 1, in dem das bioaktive Material Hydroxyapatit umfasst.

3. Knochenimplantat nach Anspruch 1 oder Anspruch 2, in dem die Ionen der Oberflächen-Atomschichten des Knochenimplantats bis zu einer maximalen Tiefe von 200 nm einverleibt werden.

4. Knochenimplantat nach Anspruch 1 oder Anspruch 2, in dem die Ionen der Oberfläche des Knochenimplantats bis zu einer maximalen Tiefe von 150 nm einverleibt werden.

5. Knochenimplantat nach Anspruch 4, in dem die Ionen der Oberfläche bei Tiefen im Bereich bis zu etwa 100 nm einverleibt werden.

6. Knochenimplantat nach irgendeinem der vorangehenden Ansprüche, in dem die Ionen in einer Konzentration von 1 x 10¹⁰ bis 1 x 10¹⁸ Ionen pro cm² Oberfläche vorliegen.

7. Knochenimplantat nach irgendeinem der vorangehenden Ansprüche, in dem die Ionen ausgewählt sind aus einer oder mehreren Gruppen des Periodensystems, die aus den Gruppen IIA, IVB, VIB, VIIB, VIII, IB, IIB, IVA und VIIA bestehen.

8. Knochenimplantat nach Anspruch 7, in dem die Ionen ein oder mehrere der folgenden umfassen:
Magnesium-, Calcium-, Strontium-, Titan-, Chrom-, Mangan-, Eisen-, Kupfer-, Zink-, Silicium- und Fluorionen.

9. Knochenimplantat nach Anspruch 7, in dem die Ionen, die der Oberfläche des Knochenimplantats einverleibt werden, aus einer oder mehreren der Gruppen des Periodensystems sind, die aus den Gruppen IIA, VIIB, IIB, IVA und VIIA bestehen.

10. Knochenimplantat nach irgendeinem der vorangehenden Ansprüche, in dem die Ionen Magnesium-, Mangan-, Zink- oder Siliciumionen umfassen.

11. Knochenimplantat nach irgendeinem der vorangehenden Ansprüche, umfassend einen Körperabschnitt, der mit einem bioaktiven Materialüberzug überzogen ist.

12. Knochenimplantat nach Anspruch 11, in dem der Körperabschnitt aus einem Metall oder einer Metall-Legierung, vorzugsweise einer Titanlegierung, gebildet ist.

13. Knochenimplantat nach irgendeinem der Ansprüche 1 bis 10, in dem das Knochenimplantat größtenteils ein bioaktives Material umfasst.

14. Knochenimplantat nach Anspruch 13, in dem das Knochenimplantat in granulärer Form vorliegt.

15. Verfahren zur Behandlung eines Knochenimplantats mit einer Oberfläche, die ein bioaktives Material umfasst, um die Knochenaufwachstums-Eigenschaften des Knochenimplantats zu verbessern, welches umfasst, dass man das Knochenimplantat einer Ionenstrahl-Einbettung unterzieht, um dadurch man Ionen aus einer oder mehreren der Gruppen des Periodensystems, die aus den Gruppen IIA, IVA, VIIA und Übergangselementen bestehen, der Oberfläche einzuverleiben.

16. Verfahren nach Anspruch 15, in dem das bioaktive Material Hydroxyapatit umfasst.

17. Vertahren nach Anspruch 15 oder Anspruch 16, in dem die Ionen der Oberfläche bis zu einer maximalen Tiefe von 200 nm einverleibt werden.

18. Verfahren nach Anspruch 17, in dem die Ionen der Oberfläche bis zu einer maximalen Tiefe von 150 nm einverleibt werden.

19. Verfahren nach Anspruch 18, in dem die Ionen bei Tiefen im Bereich bis zu etwa 100 nm einverleibt werden.

20. Verfahren nach irgendeinem der Ansprüche 14 bis 19, in dem die Ionen von 1 x 10¹⁰ bis 1 x 10¹⁸ Ionen pro cm² der Implantat-Oberfläche vorliegen.

21. Verfahren nach irgendeinem der Ansprüche 14 bis 20, in dem die Ionen ausgewählt sind aus einer oder mehreren Gruppen des Periodensystems, die aus den Gruppen IIA, IVB, VIB, VIIB, VIII, IB, IIB, IVA und VIIA bestehen.

22. Verfahren nach Anspruch 21, in dem die Ionen eine oder mehrere der folgenden umfassen:
Magnesium-, Calcium-, Strontium-, Titan-, Chrom-, Mangan-, Eisen-, Kupfer-, Zink-, Silicium- und Fluorionen.

23. Verfahren nach Anspruch 21, in dem die Ionen, die der Oberfläche des Knochenimplantats einverleibt werden, aus einer oder mehreren der Gruppen des Periodensystems ausgewählt sind, die aus den Gruppen IIA, VIIB, IIB, IVA und VIIA bestehen.

24. Verfahren nach Anspruch 23, in dem die Ionen Magnesium-, Mangan-, Zink- oder Siliciumionen umfassen.

## Revendications

1. Implant osseux ayant une surface comprenant une matière biologiquement active, dans lequel :
(a) la matière biologiquement active renferme des ions d'un ou plusieurs des groupes du Tableau Périodique consistant en les groupes IIA, IVA, VIIA et les éléments de transition ;
(b) les ions sont incorporés dans ou sur la surface de l'implant osseux par implantation par faisceau ionique ou dépôt par arc cathodique ; et
(c) la matière biologiquement active est une matière qui est capable d'activer la croissance osseuse sur l'implant osseux.

2. Implant osseux suivant la revendication 1, dans lequel la matière biologiquement active comprend l'hydroxyapatite.

3. Implant osseux suivant la revendication 1 ou la revendication 2, dans lequel les ions sont incorporés aux couches atomiques de surface de l'implant osseux jusqu'à une profondeur maximale de 200 nm.

4. Implant osseux suivant la revendication 1 ou la revendication 2, dans lequel les ions sont incorporés dans la surface de l'implant osseux jusqu'à une profondeur maximale de 150 nm.

5. Implant osseux suivant la revendication 4, dans lequel les ions sont incorporés dans la surface à des profondeurs allant jusqu'à approximativement 100 nm.

6. Implant osseux suivant l'une quelconque des revendications précédentes, dans lequel les ions sont présents en une teneur comprise dans l'intervalle de 1 x 10¹⁰ à 1 x 10¹⁸ ions par cm² de la surface.

7. Implant osseux suivant l'une quelconque des revendications précédentes, dans lequel les ions sont choisis dans un ou plusieurs groupes du Tableau Périodique, consistant en les groupes IIA, IVB, VIB, VIIB, VIII, IB, IIB, IVA et VIIA.

8. Implant osseux suivant la revendication 7, dans lequel les ions comprennent un ou plusieurs des suivants :
ions magnésium, calcium, strontium, titane, chrome, manganèse, fer, cuivre, zinc, silicium et fluor.

9. Implant osseux suivant la revendication 7, dans lequel les ions incorporés à la surface de l'implant osseux font partie d'un ou plusieurs des groupes du Tableau Périodique consistant en les groupes IIA, VIIB, IIB, IVA et VIIA.

10. Implant osseux suivant l'une quelconque des revendications précédentes, dans lequel les ions comprennent les ions magnésium, manganèse, zinc ou silicium.

11. Implant osseux suivant l'une quelconque des revendications précédentes, comprenant une partie consistant en un corps revêtu d'un revêtement de matière biologiquement active.

12. Implant osseux suivant la revendication 11, dans lequel la partie servant de corps est formée d'un métal ou d'un alliage métallique, de préférence d'un alliage de titane.

13. Implant osseux suivant l'une quelconque des revendications 1 à 10, dans lequel ledit implant osseux comprend substantiellement une matière biologiquement active.

14. Implant osseux suivant la revendication 13, dans lequel ledit implant osseux est sous forme granulaire.

15. Procédé pour le traitement d'un implant osseux ayant une surface comprenant une matière biologiquement active pour améliorer les propriétés de croissance en surface osseuse de l'implant osseux, comprenant l'étape consistant à soumettre l'implant osseux à une inclusion par faisceau ionique pour incorporer ainsi des ions d'un ou plusieurs des groupes du Tableau Périodique consistant en les groupes IIA, IVA, VIIA et les éléments de transition dans la surface.

16. Procédé suivant la revendication 15, dans lequel la matière biologiquement active comprend l'hydroxyapatite.

17. Procédé suivant l'une quelconque des revendications 15 et 16, dans lequel les ions sont incorporés dans la surface jusqu'à une profondeur maximale de 200 nm.

18. Procédé suivant la revendication 17, dans lequel les ions sont incorporés dans la surface jusqu'à une profondeur maximale de 150 nm.

19. Procédé suivant la revendication 18, dans lequel les ions sont incorporés à des profondeurs allant jusqu'à approximativement 100 nm.

20. Procédé suivant l'une quelconque des revendications 14 à 19, dans lequel les ions sont présents en une teneur comprise dans l'intervalle de 1 x 10¹⁰ à 1 x 10¹⁸ ions par cm² de la surface d'implant.

21. Procédé suivant l'une quelconque des revendications 14 à 20, dans lequel les ions sont choisis dans un ou plusieurs des groupes du Tableau Périodique consistant en les groupes IIA, IVB, VIB, VIIB, VIII, IB, IIB, IVA et VIIA.

22. Procédé suivant la revendication 21, dans lequel les ions comprennent un ou plusieurs des suivants :
ions magnésium, calcium, strontium, titane, chrome, manganèse, fer, cuivre, zinc, silicium et fluor.

23. Procédé suivant la revendication 21, dans lequel les ions incorporés à la surface de l'implant osseux font partie d'un ou plusieurs des groupes du Tableau Périodique consistant en les groupes IIA, VIIB, IIB, IVA et VIIA.

24. Procédé suivant la revendication 23, dans lequel les ions comprennent des ions magnésium, manganèse, zinc ou silicium.
